# EUROPEAN PATENT APPLICATION

(11) **EP 4 851 298 A1**
(43) Date of publication of application: **09.12.2026**
(21) Application number: 25180523.0
(22) Date of filing: 03.06.2025
(51) Int. Cl.: C12P 21/00, C07K 16/00, C12N 9/48

(54) **SUGAR CONJUGATES AND METHODS FOR THEIR PRODUCTION**

(71) Applicant: Fraunhofer-Gesellschaft zur Förderung der angewandten Forschung e.V., 80686 München (DE)
(72) Inventor: Safarian, Schara, 60596 Frankfurt am Main (DE); Rasoulinejad, Samaneh, 60596 Frankfurt am Main (DE); Kannt, Aimo, 60596 Frankfurt am Main (DE)
(74) Representative: Kuttenkeuler, David

(57) **Abstract**

The invention is based on protein-sugar complexes produced by a sortase based enzymatic reaction. The invention pertains to complexes, methods of their synthesis and uses of such complexes, for example in the production of biologically active molecules, preferably for use in medicine.

## Description

### FIELD OF THE INVENTION

The invention is based on protein-sugar complexes produced by a sortase based enzymatic reaction. The invention pertains to complexes, methods of their synthesis and uses of such complexes, for example in the production of biologically active molecules, preferably for use in medicine.

### DESCRIPTION

Glycosylation represents a fundamental post-translational modification of proteins, wherein carbohydrate moieties (glycans) are covalently attached to specific amino acid residues. This modification occurs predominantly within eukaryotic cells, in particular within the endoplasmic reticulum and Golgi apparatus, and plays a critical role in influencing protein folding, stability, solubility, cellular localization, and biological activity. Glycosylation is further recognized as a key determinant in mediating cell-cell interactions, immune responses, and protein clearance rates.

Two principal forms of glycosylation are commonly observed: N-linked and O-linked glycosylation. N-linked glycosylation involves the attachment of glycans to the nitrogen atom of asparagine residues, typically within the consensus sequence Asn-X-Ser/Thr (where X is any amino acid except proline), and is initiated in the endoplasmic reticulum with subsequent processing in the Golgi apparatus. O-linked glycosylation, by contrast, entails the attachment of glycans to the hydroxyl group of serine or threonine residues, and is generally initiated in the Golgi.

In the context of recombinant protein expression and therapeutic protein development, glycosylation is of particular relevance, as the presence, absence, or structure of glycan moieties may directly affect the pharmacological properties of a given protein, including its biological activity, in vivo half-life, and immunogenic potential. For example, the glycosylation profile of monoclonal antibodies may influence effector functions such as antibody-dependent cellular cytotoxicity (ADCC). Accordingly, the ability to control, analyze, and characterize glycosylation patterns is of critical importance in the production and regulatory approval of biopharmaceuticals. Established analytical techniques for assessing glycosylation include, but are not limited to, mass spectrometry, chromatography, and lectin-based binding assays.

Artificial glycosylation of proteins may be accomplished through a variety of established methodologies, including enzymatic, chemical, and cell-based approaches. These techniques enable the introduction, modification, or control of glycan structures on proteins for various applications, including therapeutic development, structural analysis, and functional studies.

Enzymatic glycosylation involves the use of glycosyltransferases or endoglycosidases to attach defined glycan structures to specific sites on a protein in vitro. This method allows for high specificity and reproducibility and is often employed to produce homogenous glycoforms or to remodel glycosylation patterns following protein expression. Chemical glycosylation, by contrast, utilizes site-specific or random covalent conjugation of glycans or glycan mimetics to amino acid side chains through chemically reactive groups. Techniques such as click chemistry are commonly employed in this context to enable efficient and selective glycan attachment, including the incorporation of non-natural sugar analogs.

Chemoenzymatic methods combine the precision of chemical site-modification with the selectivity of enzymatic glycan transfer, thereby offering a flexible and efficient strategy for constructing structurally defined glycoproteins. In addition, cell-based glycoengineering approaches are widely used for large-scale production of glycoproteins with specific glycosylation profiles. These methods typically involve the use of genetically modified host cells-such as Chinese hamster ovary (CHO) cells, human embryonic kidney (HEK293) cells, yeast, or engineered bacterial systems-wherein the endogenous glycosylation pathways are altered or supplemented with exogenous enzymes to produce desired glycoforms.

Despite the utility of artificial glycosylation methods, each approach presents inherent limitations. Enzymatic glycosylation is constrained by the limited availability and substrate specificity of glycosyltransferases, as well as the high cost of enzymatic reagents. Chemical glycosylation often suffers from poor site specificity and may require reaction conditions that can compromise protein integrity, while also limiting the complexity of glycan structures that can be introduced. Chemoenzymatic strategies, though more precise, involve multi-step processes that can be labor-intensive and challenging to optimize. Cell-based glycoengineering approaches, while scalable, may produce heterogeneous or non-human glycan structures, necessitating complex genetic modifications and raising regulatory concerns in therapeutic applications. These limitations underscore the need for improved glycosylation methods that are efficient, site-specific, scalable, and capable of producing biologically relevant glycoforms. Therefore, the present inventions object is to provide a more efficient and easy procedure to allow for targeted modification of target molecules with sugar structures.

### BRIEF DESCRIPTION OF THE INVENTION

The above problem of the invention is solved by way of brief description, the main aspects of the present invention, which can be described as follows:

In **a first aspect,** the invention pertains to a method adding a carbohydrate to a target polypeptide or to a target protein, the method comprising reacting an acceptor protein construct with a donor carbohydrate construct in the presence of an enzyme having sortase function, wherein
(A) The acceptor protein construct has the formula:

   **TP-L1-SRM-L2-DT**

   wherein:
   **TP** is a target polypeptide or target protein,
   **SRM** is a polypeptide having an amino acid sequence comprising a sortase recognition motif, and
   **DT** is absent or is a detectable tag,
   **L1** is absent or a first linker,
   **L2** is absent a second linker; and
(B) The donor carbohydrate construct has the formula:

   **OG-L-SM**

   wherein:
   **OG** is Xₙ with X being an amino acid, n = 1 or more; preferably X is G, A, S, T, C or K, preferably OG is an oligoglycine,
   **L** is absent or a linker
   **SM** is a sugar molecule, preferably an amino sugar molecule.

In **a second aspect,** the invention pertains to an acceptor protein construct, comprising the formula:

**TP-L1-SRM-L2-DT**

wherein:
**TP** is a target polypeptide or target protein,
**SRM** is a polypeptide having an amino acid sequence comprising a sortase recognition motif, and
**DT** is absent or is a detectable tag,
**L1** is absent or a first linker,
**L2** is absent a second linker.

In **a third aspect,** the invention pertains to a donor carbohydrate construct, comprising the formula:

**OG-L-SM**

wherein:
**OG** is Xₙ with n = 1 or more; preferably OG is an oligoglycine,
**L** is absent or a linker
**SM** is a sugar molecule, preferably an amino sugar molecule.

In a **fourth** aspect, the invention pertains a protein sugar conjugate construct, having the formula:

**TP-L1-partialSRM1-OG-L-SM**

wherein
**TP** is a target polypeptide or target protein,
**partialSRM1** is a polypeptide having an amino acid sequence comprising a N-terminal part of the sortase recognition motif (lacking an C-terminal part of the sortase recognition motif)
**L1** is absent or a first linker,
**OG** is Xₙ with n = 1 or more; preferably OG is an oligoglycine,
**L** is absent or a linker.
**SM** is a sugar molecule, preferably an amino sugar molecule.

In **a fifth aspect,** the invention pertains to a use of the donor carbohydrate construct of the previous aspects in the conjugation of a carbohydrate to a target protein or target polypeptide.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

For clarity of understanding the present invention, the definition of terms of preferred embodiments of certain components recited in individual aspects shall also equally apply to other aspects. The present disclosure of aspects are not to be read in isolation to each other.

In **a first aspect,** the invention pertains to a method adding (connecting) a carbohydrate to a target polypeptide or to a target protein, the method comprising reacting an acceptor protein construct with a donor carbohydrate construct in the presence of an enzyme having sortase function, wherein
(A) The acceptor protein construct has the formula:

   **TP-L1-SRM-L2-DT**

   wherein:
   **TP** is a target polypeptide or target protein,
   **SRM** is a polypeptide having an amino acid sequence comprising a sortase recognition motif, and
   **DT** is absent or is a detectable tag,
   **L1** is absent or a first linker,
   **L2** is absent a second linker; and
(B) The donor carbohydrate construct has the formula:

   **OG-L-SM**

   wherein:
   **OG** is Xₙ with X being an amino acid, n = 1-10 or more; preferably X is G, A, S, T, C or K, preferably OG is an oligoglycine,
   **L** is absent or a linker
   **SM** is a sugar molecule, preferably an amino sugar molecule.

In context of the present disclosure the use of the terms target protein or target polypeptide shall be understood as being synonymous and to relate to a peptide-bond based target molecule that is characterized by the presence of peptide-bond N- and C-terminus in the molecule. The target protein may be a singly molecule or a complex multimeric protein (such as an antibody).

As used herein, the term "a target protein" refers to all members of the target family, and fragments and enantiomers thereof and protein mimics thereof. The target proteins of interest that are described herein are intended to include all members of the target family, and fragments and enantiomers thereof, and protein mimics thereof, unless explicitly described otherwise. The target protein may be any protein of interest, such as a therapeutic or diagnostic target, including but not limited to: hormones, growth factors, receptors, enzymes, cytokines, osteoinductive factors, colony stimulating factors and immunoglobulins. The term "target protein" is intended to include recombinant and synthetic molecules, which can be prepared using any convenient recombinant expression methods or using any convenient synthetic methods, or purchased commercially, as well as fusion proteins containing a target molecule. Specifically preferred target proteins are antigen binding proteins such as receptors, ligans, antibodies, any antigen binding fragments thereof, VHH-antibodies, T-cell receptor based constructs, Chimeric antigen receptors (also soluble forms thereof).

The term "adding" or "connecting" shall refer to the establishment of a covalent connection between two entities based on a sortase reaction. The term shall also include the attachment of complexes that contain more than one molecular entity, but which are connected either covalently or non-covalently (affinity based). However, if such complexes are added or connected together in accordance with the present invention, the term shall in particular refer to such adding or connection which involves the formation of a covalent bond via a sortase reaction.

According to the invention the component SM refers to a carbohydrate. The carbohydrate or saccharide according to the present invention is not limited by its source or origin, and encompasses those obtained from natural origins including those from human and other mammalian sources, those produced by genetically engineered animal cells, plant cells, microorganisms, and other cells, those enzymatically manufactured, those manufactured by fermentation processes, those artificially synthesized by chemical processes and others. The carbohydrate or saccharide may encompass monosaccharides, disaccharides, oligosaccharides, and polysaccharides. Examples of N-acyl-2-amino monosaccharides include, but are not limited to, N-acetylglucosamine, N-acylglucosamine, N-acetylgalactosamine, N-acylgalactosamine, **N-**acetylmannosamine, N-acylmannosamine, N-acetyllallosamine, N-acylallosamine, (2S,3S,4R,5R,6R)-5-acetamido-3,4,6-trihydroxytetrahydro-2H-pyran-2-carboxylic acid, (2S,3R,4R,5R,6R)-5-acetamido-3,4,6-trihydroxytetrahydro-2H-pyran-2-carboxylic acid, (2S,3S,4S,5R,6R)-5-acetamido-3,4,6-trihydroxytetrahydro-2H-pyran-2-carboxylic acid, (2R,3S,4R,5R,6R)-5-acetamido-3,4,6-trihydroxytetrahydro-2H-pyran-2-carboxylic acid, (2S,3S,4R,5R,6R)-5-acyl-3,4,6-trihydroxytetrahydro-2H-pyran-2-carboxylic acid, (2S,3R,4R,5R,6R)-5-acyl-3,4,6-trihydroxytetrahydro-2H-pyran-2-carboxylic acid, (2S,3S,4S,5R,6R)-5-acyl-3,4,6-trihydroxytetrahydro-2H-pyran-2-carboxylic acid, (2R,3S,4R,5R,6R)-5-acyl-3,4,6-trihydroxytetrahydro-2H-pyran-2-carboxylic acid, (2S,3S,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2S,3R,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2S,3S,4S,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2R,3S,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, and derivatives and modified derivatives thereof. In a preferred embodiment, the monosaccharide has a molecular weight of at least about 200 Daltons to about 500 Daltons.

Examples of disaccharides include, but are not limited to, N-acetylactosamine, N-acylactosamine, N,N'-diacetyl chitobiose, N,N'-diacyl chitobiose, hyaluronic acid disaccharide, keratin disaccharide, keratin sulfate disaccharide, chondroitin disaccharide, chondroitin sulfate disaccharide, dermatan disaccharide, dermatan sulfate disaccharide, heparin disaccharide, and derivatives thereof.

The oligosaccharides of the current invention encompass those molecules composed of three or more monosaccharide units linked together at any free hydroxyl in the ordinary sense, either in a linear or branched manner, and are usually composed of 3 to 30 monosaccharide units, but in some instances may contain thousands of monosaccharide units. In particular, the oligosaccharides of the present invention may further include those oligosaccharides that are isolated or synthesized wherein any C3, C4 or C6 free hydroxyl of an N-acyl-2-amino monosaccharide is substituted with a single monosaccharide, and any other C2, C3, C4 or C6 free hydroxyl may be further substituted with one or more monosaccharides. Examples of the oligosaccharide include those discovered in a wide range of organisms such as animals, plants (including seaweeds), insects, microorganisms, and others, and include, but are not limited to, homooligomers of N-acetylglucosamine, chitin, partially deacetylated chitin, and other N-acyl-2-amino monosaccharides, heterooligomers composed of two or more different monosaccharides independently selected from the groups consisting of glucose, galactose, mannose, glucosamine, fructose, N-acyl-2-amino monosaccharides, iduronic acid, neuraminic acid, and sialic acid so long as the reducing end unit is unprotected (i.e. contains a hemiacetalic hydroxyl) and contains an N-acyl-2-amino moiety. Other representative oligosaccharides include hyaluronic acid, keratin, keratin sulfate, chondroitin, chondroitin sulfate, dermatan, dermatan sulfate, heparin, N-acetyllactosamine oligosaccharide, N-acetylchitotriose, N-acetylchitotetraose, and N-acetylchitopentose, chitin, including partially deacetylated chitin, native N-glycan cores, high-mannose N-glycans, hybrid N-glycans, complex N-glycans, and derivatives thereof.

Examples of native N-glycan cores include, but are not limited to, Man3 and Man3F. Examples of high-mannose N-glycans include, but are not limited to, Man-5, Man-6, Man-7, Man-8, and Man-9. Examples of hydrid N-glycans include, but are not limited, those carbohydrates wherein the GlcNAc terminates one branch of the N-glycan core, and a mannose terminates another branch of the N-glycan core. Examples of complex N-glycans include, but are not limited to, GO-N, GO, GOF-N, GOF, GOFB, G1, G1F, G2, G2F, G2FB, G1S1(α2,3), G1S1(α2,6), G1FS1(α2,3), G1FS1(α2,6), G2S1(α2,3), G2S1(α2,6), G2FS1(α2,3), G2FS1(α2,6), G2S2(α2,3), G2S2(α2,6), G2FS2(α2,3), G2FS2(α2,6), G2F with 2-α-Gal, A3, G3, G3S3(α2,6), A4, G4, Lewis A, Lewis B, Lewis X, Lewis Y, sialyl Lewis A, and sialyl Lewis X. The nomenclature used to describe the N-glycans would be known to one skilled in the art. With the exception of the Lewis antigens, all native N-glycan cores, high-mannose N-glycans, hybrid N-glycans, and complex N-glycans contain a Man₃GlcNAc₂ core, which may be optionally fucosylated or xylosylated.

The term "modified carbohydrate" (or "modified derivative thereof") used herein may refer to those modified through any process of isolation, separation and purification from naturallyoccurring sources and origins, those that have been enzymatically modified, those that have been chemically modified, those that have been modified by biochemical means, including microorganisms, wherein such modifications may comprise those known in the field of glycoscience, for example, hydrolysis, oxidation, reduction, esterification, acylation, amination, etherification, nitration, dehydration, glycosylation, phosphorylation, and sulfation.

Linear oligosaccharides such as chitin, including partially deacetylated chitin, hyaluronic acid, keratin, keratin sulfate, chondroitin, chondroitin sulfate, dermatan, dermatan sulfate, and heparin can achieve molecular weights as much as 5,000,000 Daltons. Preferred sizes of these oligosaccharides include, but are not limited to, those with molecular weights of at least about 500 Daltons, at least about 1,000 Daltons, at least about 1,500 Daltons, at least about 3,500 Daltons, at least about 5,000 Daltons, at least about 10,000 Daltons, at least about 20,000 Daltons, at least about 25,000 Daltons, at least about 33,000 Daltons, at least about 50,000 Daltons, and at least about 100,000 Daltons. In another embodiment, preferred sizes of these oligosaccharides include, but are not limited to, those molecular weights that are less than about 6,000 Daltons, and less than about 50,000 Daltons.

Branched oligosaccharides such as high-mannose N-glycans, hybrid N-glycans, and complex N-glycans have molecular weights that are typically under 10,000 Daltons. Preferred sizes of these oligosaccharides include, but are not limited to, those with molecular weights of from about 500 Daltons to about 10,000 Daltons. A more preferred size of these oligosaccharides is from about 800 Daltons to about 5,000 Daltons. A most preferred size of these oligosaccharides is from about 800 Daltons to about 4500 Daltons.

A preferred SM is GalNac, preferably TriGalNac.

In some embodiments, a recognition sequence of a sortase further comprises one or more additional amino acids, e.g., at the N or C terminus. For example, one or more amino acids (e.g., up to 5 amino acids) having the identity of amino acids found immediately N-terminal to, or C-terminal to, a 5 amino acid recognition sequence in a naturally occurring sortase substrate may be incorporated. Such additional amino acids may provide context that improves the recognition of the recognition motif.

Sortases have been classified into 4 classes, designated A, B, C, and D, based on sequence alignment and phylogenetic analysis of 61 sortases from Gram positive bacterial genomes (Dramsi S, Trieu-Cuot P, Bieme H, Sorting sortases: a nomenclature proposal for the various sortases of Gram-positive bacteria. Res Microbiol. 156(3):289-97, 2005. These classes correspond to the following subfamilies, into which sortases have also been classified by Comfort and Clubb (Comfort D, Clubb R T. A comparative genome analysis identifies distinct sorting pathways in gram-positive bacteria. Infect Immun., 72(5):2710-22, 2004): Class A (Subfamily 1), Class B (Subfamily 2), Class C (Subfamily 3), Class D (Subfamilies 4 and 5). The aforementioned references disclose numerous sortases and recognition motifs. See also Pallen, M. J.; Lam, A. C.; Antonio, M.; Dunbar, K. TRENDS in Microbiology, 2001, 9(3), 97- 101. Those skilled in the art will readily be able to assign a sortase to the correct class based on its sequence and/or other characteristics such as those described in Drami, et al., supra. The term "sortaseA" is used herein to refer to a class Asortase, usually named SrtA in any particular bacterial species, e.g., SrtA from S. aureus. Likewise, "sortase B" is used herein to refer to a class B sortase, usually named SrtB in any particular bacterial species, e.g., SrtB from S. aureus.

A sortase to be used in context of the invention can be preferably selected from an improved sortase as published in Chen, L., Cohen, J., Song, X. et al. Improved variants of SrtA for site-specific conjugation on antibodies and proteins with high efficiency. Sci Rep 6, 31899

(2016). https://doi.org/10.1038/srep31899 (incorporated by reference herein in its entirety). A preferred sortase, which was used in context of the present invention, is 5M/D124G of that reference.

In context of the invention a Sortase Recognition Motif is denoted as "LPXTG". The position of "X" in the **LPXTG motif** is considered variable because this residue does not play a critical role in the cleavage or anchoring process facilitated by Sortase A. Instead, Sortase A primarily interacts with the conserved residues, especially the leucine (L), proline (P), threonine (T), and glycine (G) of the motif. Structurally, the side chain of "X" faces away from the active site pocket of Sortase A, meaning it has limited contact with the enzyme. This allows Sortase A to tolerate a wide range of amino acids at this position without significantly affecting enzymatic activity. However, the nature of "X" can influence the local structure of the substrate protein, potentially affecting cleavage efficiency in some cases. The following **Table 1** summarizes certain variants of the SRM according to the invention

**Table 1: Variants of Sortase Recognition Motifs (SRMs)**

| | | Preferred nucleophile |
|---|---|---|
| Sp-Sortase A as wild type Cd-Sortase A | LPXTG (SEQ ID NO.1) | N-Gly-Gly |
| | LPXLG (SEQ ID NO.2) | N-Ala-Ala |
| | LPLTG (SEQ ID NO.3) | Internal Lys of SpaA |
| | NPQTN (SEQ ID NO.4) | |
| | QVPTG (SEQ ID NO.5) | |
| | LPNTA (SEQ ID NO.6) | |
| | LPXTG/LPXTA (SEQ ID NO.1/7) | |
| | LPXTG (SEQ ID NO.1) | |
| | LAXTG/LPXTG (SEQ ID NO.8/1) | |
| | LPLTG (SEQ ID NO.9) | |
| Mutant with enhanced activity | LPXTG (SEQ ID NO.1) | N-Gly-Gly |
| | LPEXG( X=A;C;S) (SEQ ID NO.10) | |
| | LAETG (SEQ ID NO.11) | |
| Sortase mutant with altered specificity | APXTG (SEQ ID NO.12) | N-Gly-Gly |
| | FPXTG (SEQ ID NO.13) | |
| | LPXSG (SEQ ID NO.14) | |
| | LAXTG (SEQ ID NO.8) | |
| | LPXTG (SEQ ID NO.15) | |
| | NPQTN (SEQ ID NO.16) | |
| | XPKTG(X=A;D;S) (SEQ ID NO.17) | |
| | APATG/FPXTG (SEQ ID NO.18/19) | |
| | LAETG (SEQ ID NO.20) | |
| | LPEXG(X=A;C;S) (SEQ ID NO.10) | |
| | LMVGG (SEQ ID NO.21) | |

| | | |
|---|---|---|
| Ref. Engineered Sortases in Peptide and Protein Chemistry. doi.org/10.1002/cbic.202000745; And, Challenges in the use of sortase and other peptide ligases for site-specific protein modification, DOI: 10.1039/D0CS01148G | | |

In some embodiments, the sortase used for producing the conjugates described herein is a sortase A (SrtA). SrtA recognizes the motif LPXTG (SEQ ID NO.1), with common recognition motifs being, e.g., LPKTG, LPATG, LPNTG (SEQ ID NOs.22-24). In some embodiments LPETG (SEQ ID NO.25) is used. However, motifs falling outside this consensus may also be recognized. For example, in some embodiments the motif comprises an 'A' rather than a 'T' at position 4, e.g., LPXAG (SEQ ID NO.26), e.g., LPNAG (SEQ ID NO.27). In some embodiments the motif comprises an 'A' rather than a 'G' at position 5, e.g., LPXTA (SEQ ID NO.28), e.g., LPNTA (SEQ ID NO.29). In some embodiments the motif comprises a 'G' rather than 'P' at position 2, e.g., LGXTG (SEQ ID NO.30), e.g., LGATG (SEQ ID NO. 31). In some embodiments the motif comprises an T rather than 'L' at position 1, e.g., TPXTG (SEQ ID NO.32), e.g., TPNTG (SEQ ID NO.33) or TPETG (SEQ ID NO.34).

In some embodiments, the sortase used for producing the conjugates described herein is sortase B (SrtB), e.g., a sortase B of S. aureus, B. anthracis, or L. monocytogenes. Motifs recognized by sortases of the B class (SrtB) often fall within the consensus sequences NPXTX, e.g., NP[Q/K]-[T/s]-[N/G/s], such as NPQTN (SEQ ID NO.16) or NPKTG (SEQ ID NO.35). For example, sortase B of S. aureus or B. anthracis cleaves the NPQTN (SEQ ID NO.16) or NPKTG (SEQ ID NO.35) motif of IsdC in the respective bacteria (see, e.g., Marraffini, L. and Schneewind, O., Journal of Bacteriology, 189(17), p. 6425-6436, 2007). Other recognition motifs found in putative substrates of class B sortases are NSKTA (SEQ ID NO.36), NPQTG (SEQ ID NO.37), NAKTN (SEQ ID NO.38), and NPQSS (SEQ ID NO.39). For example, SrtB from L. monocytogenes recognizes certain motifs lacking P at position 2 and/or lacking Q or K at position 3, such as NAKTN (SEQ ID NO.38) and NPQSS (SEQ ID NO.39) (Mariscotti J F, Garcia-Del Portillo F, Pucciarelli M G. The listeria monocytogenes sortase-B recognizes varied amino acids at position two of the sorting motif. J Biol Chem. 2009 Jan. 7. [Epub ahead of print])

In some embodiments, the sortase used for producing the conjugates described herein is class C sortase. Class C sortases may utilize LPXTG (SEQ ID NO.1) as a recognition motif.

In some embodiments, the sortase is a class D sortase. Sortases in this class are predicted to recognize motifs with a consensus sequence NA-[E/A/S/H]-TG (SEQ ID NO.40) (Comfort D, supra). Class D sortases have been found, e.g., in Streptomyces spp., Corynebacterium spp., Tropheryma whipplei, Thermobifida fusca, and Bifidobacterium longhum. LPXTA(SEQ ID NO.28) or LAXTG (SEQ ID NO.8) may serve as a recognition sequence for class D sortases, e.g., of subfamilies 4 and 5, respectively subfamily-4 and subfamily-5 enzymes process the motifs LPXTA (SEQ ID NO.28) and LAXTG (SEQ ID NO.8), respectively). For example, B. anthracis Sortase C, which is a class D sortase, has been shown to specifically cleave the LPNTA motif in B. anthracis Basl and BasH (Marrafini, supra).

In some embodiments, a variant of a naturally occurring sortase may be used. Such variants may be produced through processes such as directed evolution, site-specific modification, etc. For example, variants of S. aureus sortase A with up to a 140-fold increase in LPETG-coupling activity compared with the starting wild-type enzyme have been identified (Chen, I., et al., PNAS 108(28): 11399-11404, 2011). In some embodiments a sortase variant comprises any one or more of the following substitutions relative to a wild type S. aureus SrtA: P94S or P94R, D160N, D165A, K190E, and K196T mutations. An exemplary wild type S. aureus SrtA sequence (Gene ID: 1125243, NCBI RefSeq Acc. No. NP_375640) is shown below:

MKKWTNRLMTIAGVVLILVAAYLFAKPHIDNYLHDKDKDEKIEQYDKNVKEQASKDNKQQAKPQI
PKDKSKVAGYIEIPDADIKEPVYPGPATPEQLNRGVSFAEENESLDDQNISIAGHTFIDRPNYQFT
NLKAAKKGSMVYFKVGNETRKYKMTSIRDVKPTDVEVLDEQKGKDKQLTLITCDDYNEKTGVW
EKRKIFVATEVK (SEQ ID NO.41)

In some embodiments, the sortase recognition sequence is at the N-terminus of the target protein. In some embodiments, the sortase recognition sequence is at the C-terminus of the target protein.

The component "SRM" of the compounds of the invention shall refer to a peptide sequence which comprises a sortase recognition motif as described herein above. The SRM may include additional N- and/or C-terminal amino acid positions. IN preferred embodiments, the SRM consists of (essentially) of the sortase recognition motif.

The term "detectable tag" shall refer to any molecule that mediates detection and/or purification of its tagged parent molecule. In preferred embodiments the detectable tag is a purification tag. The term "purification tag" as used herein refers to any peptide sequence suitable for purification or identification of a proteinaceous binding molecule. The purification tag specifically binds to another moiety with affinity for the purification tag. Such moieties which specifically bind to a purification tag are usually attached to a matrix or a resin, such as agarose beads. Moieties which specifically bind to purification tags include antibodies, nickel or cobalt ions or resins, biotin, amylose, maltose, and cyclodextrin. Exemplary purification tags include histidine tags (such as a hexahistidine peptide), which will bind to metal ions such as nickel or cobalt ions. Therefore, in certain embodiments the purification tag comprises a peptide sequence which specifically binds metal ions. In other embodiments the purification tag consists of a peptide sequence which specifically binds metal ions. In preferred embodiments the purification tag comprises or consists of a peptide sequence which specifically binds nickel or cobalt ions. In other embodiments, the purification tag comprises or consists of a peptide sequence which specifically binds nickel ions. In alternative embodiments, the purification tag comprises or consists of a peptide sequence which specifically binds cobalt ions. Other exemplary purification tags are the myc tag (SEQ ID NO.42: EQKLISEEDL), the Strep tag (SEQ ID NO.43: WSHPQFEK), the Flag tag (SEQ ID NO.44: DYKDDDDK) and the V5 tag (SEQ ID NO.45: GKPIPNPLLGLDST). The term "purification tag" also includes "epitope tags", i.e. peptide sequences which are specifically recognized by antibodies. Exemplary epitope tags include the FLAG tag, which is specifically recognized by a monoclonal anti-FLAG antibody. The peptide sequence recognized by the anti-FLAG antibody consists of the sequence DYKDDDDK (SEQ ID NO.44) or a substantially identical variant thereof. Therefore, in certain embodiments the purification tag comprises or consists of a peptide sequence which is specifically recognized by an antibody. The term "purification tag" also includes substantially identical variants of purification tags. "Substantially identical variant" as used herein refers to derivatives or fragments of purification tags which are modified compared to the original purification tag (e.g. via amino acid substitutions, deletions or insertions), but which retain the property of the purification tag of specifically binding to a moiety which specifically recognizes the purification tag. The purification tag may comprise or consist of a peptide sequence of less than four, four, five, six, seven, eight, nine, ten, eleven, twelve, thirteen, fourteen, fifteen, sixteen, seventeen, eighteen, nineteen, twenty, or many than twenty amino acids. In certain embodiments the purification tag comprises or consists of between four and twenty, between five and twenty, between six and twenty, between four and fifteen, between five and fifteen, between six and fifteen, between four and twelve, between five and twelve or between six and twelve amino acids.

In some embodiments it is preferred that DT is a protein tag or a fluorescent molecule tag, preferably wherein the protein tag is selected from a chitin binding protein (CBP)-tag, maltose binding protein (MBP)-tag, glutathione-S-transferase (GST)-tag, poly(His)-tag , FLAG tag, Epitope tags, such as, V5-tag, c-myc-tag, and HA-tag, and fluorescence protein tags such as green fluorescent protein (GFP), red fluorescent protein (RFP), yellow fluorescent protein (YFP), blue fluorescent protein (BFP), and cyan fluorescent protein (CFP).

A "linker" in context of the invention may be selected from a bond, an atom, or any molecule that is capable of connecting covalently or non-covalently two or more entities. A linker in some embodiments is absent, which shall in this context refer to an embodiment where two or more entities are connected without intermediary. A linker may preferably be selected from a molecule such as an amino acid (such as lysine). The person of skill is aware of specific linker molecules or peptides. L1 and L1 for example may be peptide liners. In preferred embodiments throughout the present disclosure, L1 is a GS linker preferably having the sequence GGSGGGSG (SEQ ID NO.46); and/or L is a GS linker preferably having the sequence GGGGSGGGGS (SEQ ID NO.47).

The component OG is a polyaminoacid, preferably wherein X is one type of amino acid. In some embodiments, N is 1 to 20, preferably 1 to 10, even more preferably 1-5, most preferably about 5. In some embodiments it is preferred that X is glycine, more preferably an oligo-glycine with n =3 to 7, preferably about 5.

A "partial SRM" is referring to a peptide sequence that comprises an incomplete sortase recognition site. Preferably the partial SRM consists of an incomplete (misses at least one amino acid of the sortase recognition site) sortase recognition site. Therefore, in some embodiments, the partialSRM and its surrounding sequences do not contain a complete sortase recognition site, most preferably the full construct that comprises a partialSRM does not comprise an active sortase recognition site.

In some embodiments, the **SRM** is connected to the C-terminus of **TP** directly or via **L1.**

In some embodiments, the **DT** is connected to the C-terminus of **SRM** directly or via **L2.**

In some embodiments, the **L1** and the **L2** are identical or different.

In some embodiments, the linker is a protein linker, preferably a peptide linker, more preferably a GS repeat.

In some embodiments, the sortase recognition motif is LPX₁TX₂, wherein each instance of X independently represents any amino acid residue, such as E or G, more preferably wherein X₁ is E and X₂ is G.

In a preferred embodiment, the target polypeptide or target protein is an antigen binding protein (ABP), wherein the ABP is any protein capable of binding specifically to, or unspecifically to, any other protein or polypeptide, such as preferably an antibody, or an antigen binding fragment thereof, nanobody, or an antigen binding fragment thereof, a soluble or membrane bound receptor, or a protein ligand. Preferably the target protein, may be an antibody heavy or light chain.

In yet another preferred embodiment, n in Xₙ is 2 or more, preferably is 3, 4, 5, 6, 7, or 8.

In yet another preferred embodiment the linker is a bond, or a molecular linker, preferably a single amino acid linker, or a peptide or non-peptide linker, and most preferably is Lysine.

In yet another preferred embodiment the sugar molecule is an amino sugar, such as GalNac, and preferably is TriGalNac.

In yet another preferred embodiment, the sugar is a glycan, preferably a glycan known to be present on a glycoprotein, for example the glycan may be a monosaccharide, an oligosaccharide or a polysaccharide.

In yet another preferred embodiment, the monosaccharide glycan is composed of a single N-acetylglucosamine (GlcNAc), glucose (Glc), mannose (Man) or fucose (Fuc)

In yet another preferred embodiment, the glycan when an oligosaccharide is linear or branched,

In yet another preferred embodiment, the glycan may be an O-linked glycan, or an N-linked glycan or a C-linked glycan.

In yet another preferred embodiment, the **SM** is connected to the C-terminus of **OG** directly or via a linker.

In yet another preferred embodiment, the reaction is a sortase reaction, preferably in the presence of Ca2+.

Preferably, the present invention also pertains to the disclosed method, wherein a first product of the reaction has the formula:

**TP-L1-partialSRM1-OG-L-SM**

wherein
**partialSRM1** is a N-terminal sequence of the original sortase recognition motif (which lacks an original C-terminal part of the sortase recognition motif)

The method of the invention in another preferred embodiment produces a second (side) product of the reaction has the formula:

**partialSRM2-L2-DT**

wherein
**partialSRM2** is a C-terminal sequence of the original sortase recognition motif (which lacks a N-terminal sequence of the sortase recognition motif).

In **a second aspect,** the invention pertains to an acceptor protein construct, comprising the formula:

**TP-L1-SRM-L2-DT**

wherein:
**TP** is a target polypeptide or target protein,
**SRM** is a polypeptide having an amino acid sequence comprising a sortase recognition motif, and
**DT** is absent or is a detectable tag,
**L1** is absent or a first linker,
**L2** is absent a second linker.

The definitions as provided above for the first aspect apply also for the second aspect.

In a **third aspect,** the invention pertains to a donor carbohydrate construct, comprising the formula:

**OG-L-SM**

wherein:
**OG** is Xₙ with n = 1 or more; preferably OG is an oligoglycine,
**L** is absent or a linker
**SM** is a sugar molecule, preferably an amino sugar molecule.

The definitions as provided above for the first aspect apply also for the second aspect.

An example of a donor carbohydrate construct is a compound having the following structure:

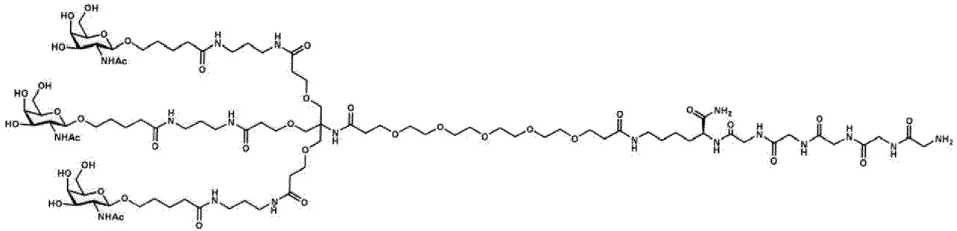

In a fourth aspect, the invention pertains a protein sugar conjugate construct, having the formula:

**TP-L1-partialSRM-OG-L-SM**

wherein
**TP** is a target polypeptide or target protein,
**partialSRM** is a polypeptide having an amino acid sequence comprising a N-terminal part of the sortase recognition motif (lacking an C-terminal part of the sortase recognition motif)
**L1** is absent or a first linker,
**OG** is Xₙ with n = 1 or more; preferably OG is an oligoglycine,
**L** is absent or a linker.
**SM** is a sugar molecule, preferably an amino sugar molecule.

The protein sugar conjugate of this aspect may in some preferred embodiments provide that the L1 is a protein linker, preferably a peptide linker, more preferably a GS repeat linker (as defined herein above).

In a preferred embodiment the sortase recognition motif is LPX₁TX₂, wherein each instance of X independently represents any amino acid residue, such as E or G, more preferably wherein X₁ is E and X₂ is G.

In another embodiment, the target polypeptide or target protein is an antigen binding protein (ABP), wherein the ABP is any protein capable of binding specifically to, or unspecifically to, any other protein or polypeptide, such as preferably an antibody, or an antigen binding fragment thereof, nanobody, or an antigen binding fragment thereof, a soluble or membrane bound receptor, or a protein ligand.

In another embodiment, the target polypeptide or target protein is a therapeutic antigen binding protein (ABP). Therapeutic antibodies are well known. Most known oncological active antibodies include trastuzumab (anti-HER2), bevacizumab (anti-VEGF), rituximab (anti-CD20), cetuximab (anti-EGFR), daratumumab (anti-CD38), and checkpoint inhibitors such as atezolizumab (anti-PD-L1), nivolumab and pembrolizumab (both anti-PD-1), and ipilimumab (anti-CTLA-4). Additional oncology agents include inotuzumab ozogamicin, an anti-CD22 antibodydrug conjugate. For autoimmune and inflammatory disorders, important INNs include adalimumab and infliximab (both anti-TNF), tocilizumab (anti-IL-6 receptor), ustekinumab (anti-IL-12/23), secukinumab (anti-IL-17A), dupilumab (anti-IL-4Rα), and belimumab (anti-BLyS). In the field of infectious diseases, antibodies such as palivizumab (anti-RSV), sotrovimab and regdanvimab (both targeting SARS-CoV-2) have been used. Other therapeutic antibodies of note include erenumab (anti-CGRP receptor) for migraine prevention, omalizumab (anti-IgE) for asthma and urticaria, and ranibizumab (anti-VEGF-A) for the treatment of age-related macular degeneration. Another example of an antibody is siltuximab, which is used also exemplary herein.

In a **fifth aspect,** the invention pertains to a use of the donor carbohydrate construct of the previous aspects in the conjugation of a carbohydrate to a target protein or target polypeptide.

The use of the invention may further include the use of a sortase enzyme as defined herein.

Another aspect relates to a kit. Preferably the kit is for performing a method of the present invention and may comprise the donor carbohydrate construct described herein, and, optionally, instruction for using the donor carbohydrate construct in a method of the invention.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

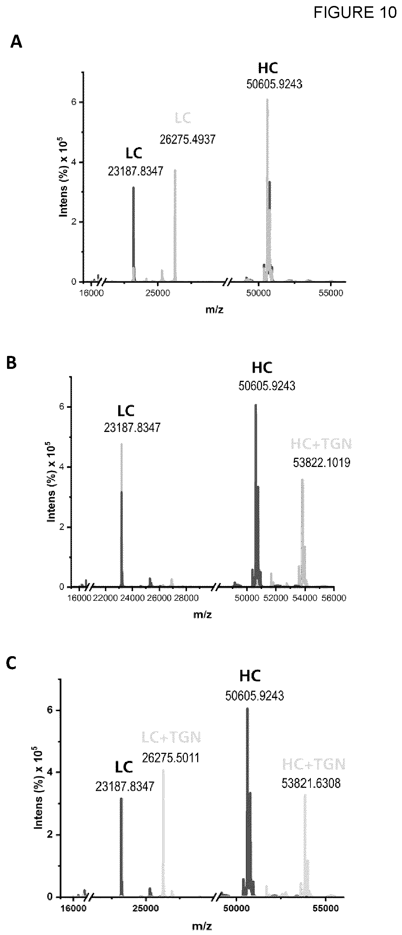

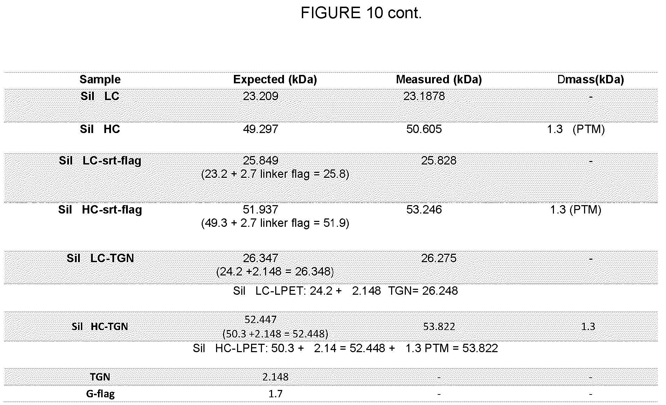

The figures show:

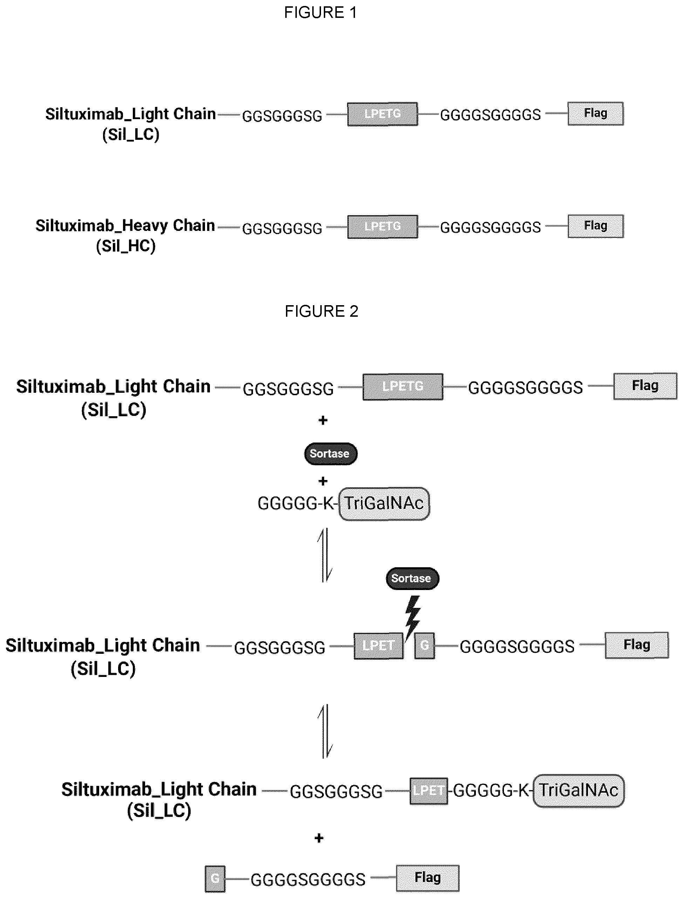

**Figure 1****:** shows two acceptor constructs of the invention. The target protein is in this case a siltuximab light chain (top sketch) and siltuximab heavy chain (bottom sketch), LPETG as a sortase motif and a FLAG tag.
**Figure 2:** shows the reaction for attaching the donor construct (wherein SM is a TriGalNac) to the acceptor constructs.

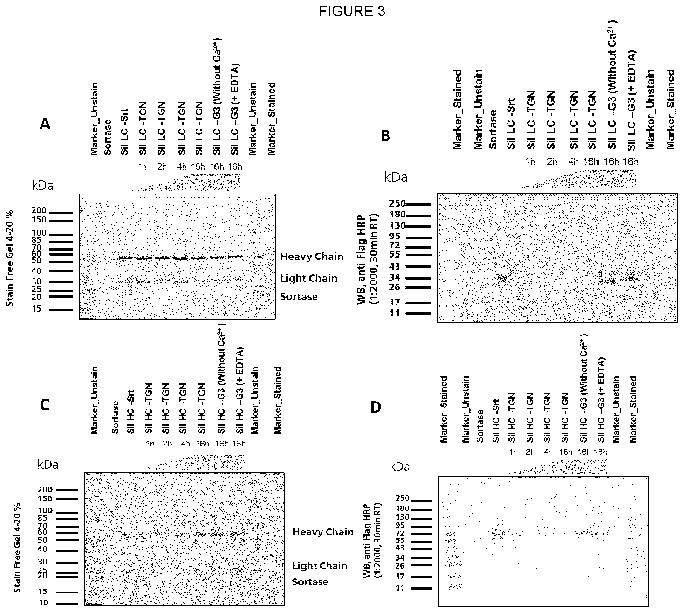

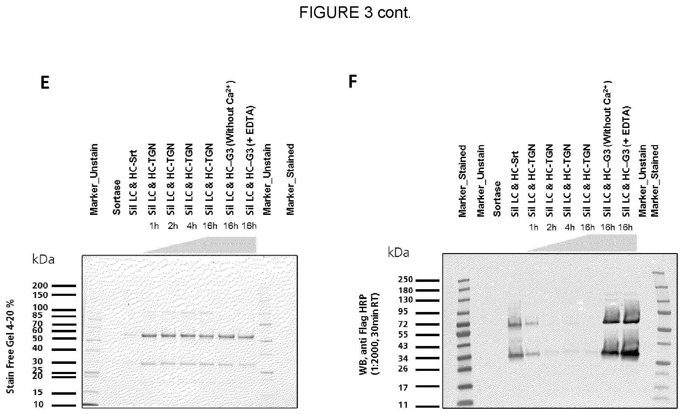

**Figure 3****:** shows the time course experiments of sortase reactions for the addition of TriGalNAc groups. Total protein staining (A, C, E) and Western-blot analyses (B, D, F) illustrate the exchange of the C-terminal detection tag during the reaction. Sil LC-Srt, Sil Hc-Srt, and Sil Lc & Hc-Srt represent unreacted reference molecules. TGN = TriGalNAc moiety. Control experiments in the absence of Ca²⁺ or in the presence of EDTA underscore the sortase-specific effects observed.

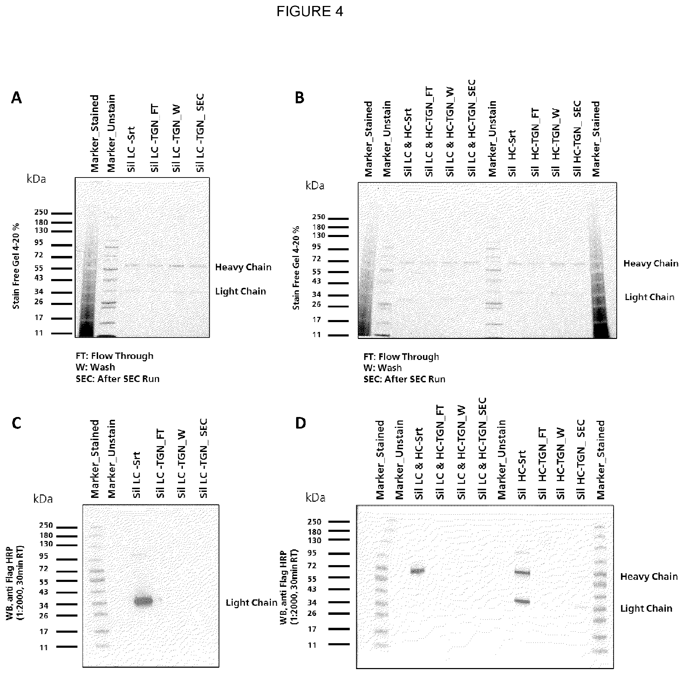

**Figure 4****:** shows the sample polishing via size exclusion chromatography and reverse affinity purification. Modified antibody samples were subjected to size exclusion chromatography using a Superdex 200 10/30 increase column to separate sortase enzyme fraction and excess donor molecules from the antibody-containing fraction. Subsequently, reverse affinity chromatography was performed using an M2 magnetic beads to separate unreacted and tagcarrying original molecules from TriGalNAc-modified variants. The flowthrough and wash fractions of the reverse affinity steps contain the polished product. Samples were analyzed by total protein staining (A, C) and Western-blot analysis. SEC = size exclusion chromatography; FT = flowthrough; W= wash fraction.

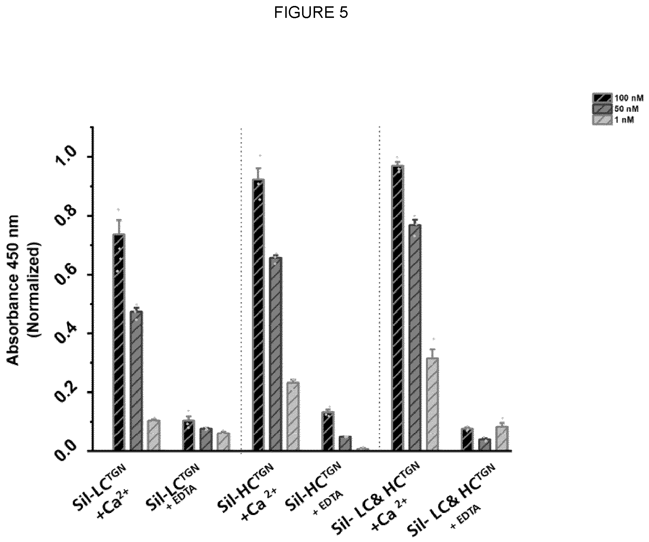

**Figure 5****:** shows the normalized ELISA results highlighting the relative binding differences of GalNAc groups in respect to their conjugation site. The experiment involved the use of a GalNAc specific cell-surface asialoglycoprotein receptor (ASGPR) to which our successfully labeled IgG-TGN variants should bind. Our three different TGN-modified IgG variants were assed for binding in an indirect ELISA setup. Data from conditions including EDTA, which chelates calcium ions necessary for glycan binding to the ASGPR, indicate strongly for a glycan-specific binding of the IgG molecules to ASGPR.

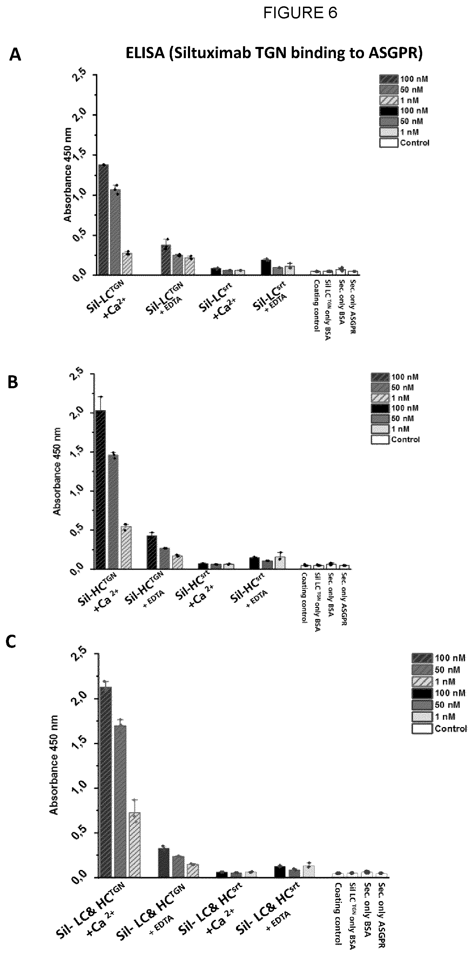

**Figure 6****:** shows the indirect ELISA conducted to functionally validate glycan labeling of IgGs. This experiment involved the use of a GalNAc specific cell-surface asialoglycoprotein receptor (ASGPR) to which our successfully labeled IgG-TGN variants should bind. Our three different TGN-modified IgG variants were assed for binding in an indirect ELISAsetup. Data from conditions including EDTA, which chelates calcium ions necessary for glycan binding to the ASGPR, indicate strongly for a glycan-specific binding of the IgG molecules to ASGPR. Additional controls included unlabeled proteins and other negative controls to confirm that the observed signal resulted from the interaction between the labeled proteins and their target. This analysis verified that the labeled proteins were functional and specifically bound to GalNAc-specific lectins.

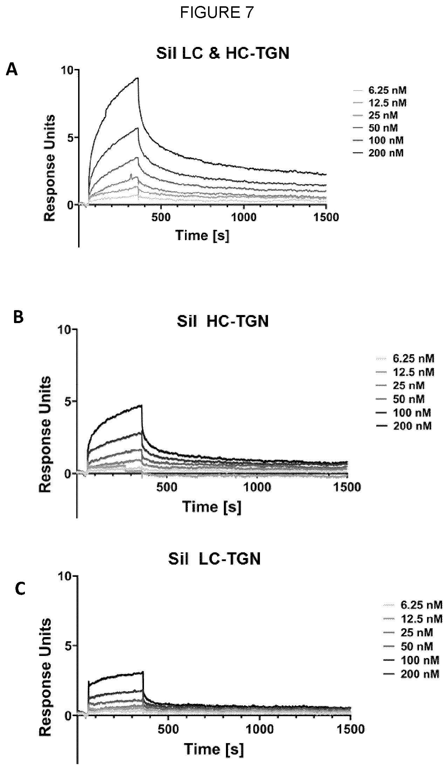

**Figure 7****:** shows the surface plasmon resonance (SPR) experiments were performed in a coupling setup using an CM5 chips and ASGPR as ligand. We evaluated kinetic properties of our three glycan-modified IgG variants (analytes) for ASGPR binding in a concentration dependent manner from 6.25 to 200 nM. The results show that higher-order modifications result in an increase of response units. Our SPR data is in well agreement with the results obtained from ELISA, indicating that labeling both the heavy chain and light chain, as well as the heavy chain alone, results in better efficiency and binding to the target compared to labeling only the light chain.

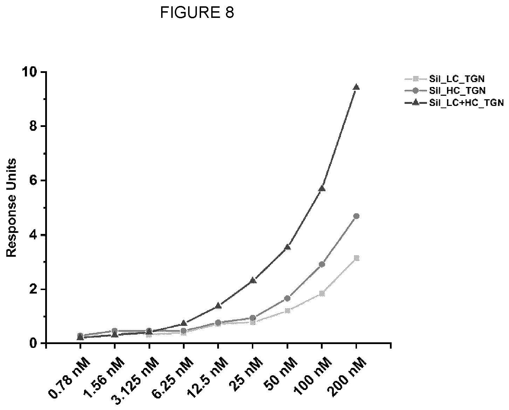

**Figure 8****:** shows the correlation between glycan-modified IgG variant concentrations and resulting response units of ASGPR-binding from SPR sensograms. The plot demonstrates that the binding of the labeled proteins to the ASGPR is concentration-dependent. It also provides a visual representation to show that labeling both the heavy and light chains, as well as the heavy chain alone, is more efficient than labeling only the light chain. This additional representation offers a clearer understanding of the data.

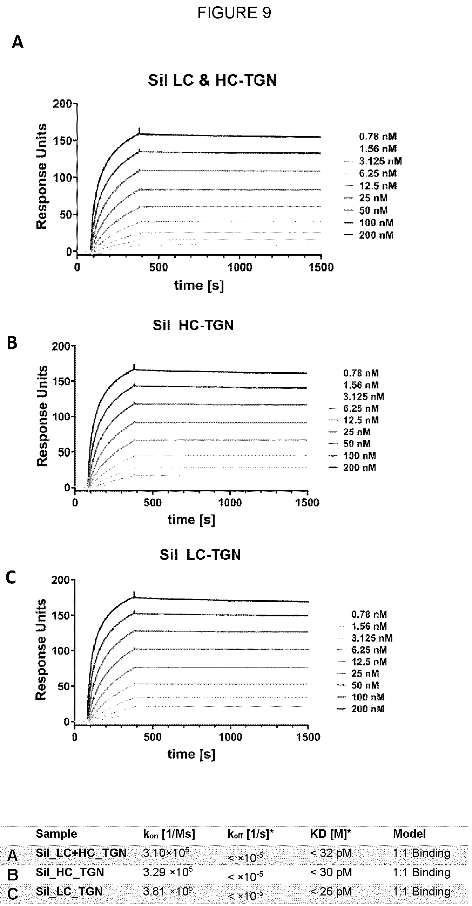

**Figure 9****:** shows the SPR experiments were performed in a coupling setup using an CM5 chips and IL6 as ligand. The experiment aimed to confirm that the affinity of the IgG variants toward the specific target remained unchanged, in this case binding of the Siltuximab toward iL-6. The analysis involved a range of labeled protein concentrations from 0.78 nM to 200 nM. The SPR data show that all labeled antibodies retained their ability to bind to the target (iL-6) with high affinity. This critical finding demonstrates that the modification process did not significantly alter the antibodies' binding characteristics to the target, ensuring the preservation of its therapeutic potential.
   asterix: (*) kd and KD weren't calculated properly due to the slow dissociation
**Figure 10****:** shows the Liquid chromatography (LC) mass spectra (MS) show the specific mass shifts as a result of sortase-mediated glycan addition to reference antibodies. The respective unlabeled IgG variants, used as a reference, are indicated in dark grey, while curves in light represents modified IgG variants. The corresponding table provides the precise molecular masses obtained by LC-MS in reference to calculated masses. As shown in the figure, there is an expected increase in size due to labeling for each site, confirming the successful modification of the protein.

The sequences show:
**SEQ ID NOs. 1 to 40 show variants of Sortase Recognition Motifs (SRMs), wherein residue denoted as x comprise any one of the naturally occurring amino acids unless otherwise specified**
   SEQ ID NO. 1: LPXTG
   SEQ ID NO. 2: LPXLG
   SEQ ID NO. 3: LPLTG
   SEQ ID NO. 4: NPQTN
   SEQ ID NO. 5: QVPTG
   SEQ ID NO. 6: LPNTA
   SEQ ID NO. 7: LPXTA
   SEQ ID NO. 8: LAXTG
   SEQ ID NO. 9: LPLTG
   SEQ ID NO. 10: LPEXG (wherein X is selected from A, C, S)
   SEQ ID NO. 11: LAETG
   SEQ ID NO. 12: APXTG
   SEQ ID NO. 13: FPXTG
   SEQ ID NO. 14: LPXSG
   SEQ ID NO. 15: LPXTG
   SEQ ID NO. 16: NPQTN
   SEQ ID NO. 17: XPKTG (wherein X is selected from A, D, S)
   SEQ ID NO. 18: APATG
   SEQ ID NO. 19: FPXTG
   SEQ ID NO. 20: LAETG
   SEQ ID NO. 21: LMVGG
   SEQ ID NO. 22: LPKTG
   SEQ ID NO. 23: LPATG
   SEQ ID NO. 24: LPNTG
   SEQ ID NO. 25: LPETG
   SEQ ID NO. 26: LPXAG
   SEQ ID NO. 27: LPNAG
   SEQ ID NO. 28: LPXTA
   SEQ ID NO. 29: LPNTA
   SEQ ID NO. 30: LGXTG
   SEQ ID NO. 31: LGATG
   SEQ ID NO. 32: TPXTG
   SEQ ID NO. 33: TPNTG
   SEQ ID NO. 34: TPETG
   SEQ ID NO. 35: NPKTG
   SEQ ID NO. 36: NSKTA
   SEQ ID NO. 37: NPQTG
   SEQ ID NO. 38: NAKTN
   SEQ ID NO. 39: NPQSS
SEQ ID NO. 40: NAXTG (x wherein X is selected from E, A, S, H) **SEQ ID NO. 41 shows an exemplary Wildtype S.aureus SrtA**
   SEQ ID NO. 41: 
MKKWTNRLMTIAGVVLILVAAYLFAKPHIDNYLHDKDKDEKIEQYDKNVKEQASKDNKQQAKPQI
PKDKSKVAGYIEIPDADIKEPVYPGPATPEQLNRGVSFAEENESLDDQNISIAGHTFIDRPNYQFT
NLKAAKKGSMVYFKVGNETRKYKMTSIRDVKPTDVEVLDEQKGKDKQLTLITCDDYNEKTGVW
EKRKIFVATEVK
**SEQ ID NO. 42 shows myc tag** SEQ ID NO. 42: EQKLISEEDL
**SEQ ID NO. 43 shows Strep tag** SEQ ID NO. 43: WSHPQFEK
**SEQ ID NO. 44 shows Flag tag** SEQ ID NO. 44: DYKDDDDK
**SEQ ID NOs. 45 shows V5 tag** SEQ ID NO. 45: GKPIPNPLLGLDST
**SEQ ID NOs. 46-47 show GS linkers:**
   SEQ ID NO. 46: GGSGGGSG
   SEQ ID NO. 47: GGGGSGGGGS

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1:

To further demonstrate the enhanced performance and advantages of the present invention, a series of experimental tests were conducted on prototypes of the novel sortase-mediated glycan labeling. The key results from these experiments are briefly described below and are further supported by the accompanying figures.

Figure 1 illustrates various design examples of acceptor polypeptides, providing a foundation for site-specific labeling using sortase. These designs highlight exemplary antibody variants specifically engineered for sortase-mediated transpeptidation, comprising a targetbinding protein, a linker, and a sortase recognition and cleavage motif. This foundational design underpins the precise targeting of glycan modification. Figure 2 then schematically illustrates the reaction mechanism for conjugating glycans to the C-termini of acceptor proteins. This figure demonstrates how the active site cysteine of sortase cleaves between specific residues, generating a thioester-linked acyl enzyme intermediate, which is subsequently attacked by an oligoglycine nucleophile conjugated with glycan residues. This process facilitates the site-specific conjugation of the peptide-linked glycan.

Figure 3 presents the results of SDS-PAGE and Western Blot analyses, which were used to assess the protein labeling efficiency of the sortase-mediated C-terminal glycan-labeling on the light chain (LC), heavy chain (HC), and both antibody chains (LC&HC) of a prototypical IgG. The Western Blot analysis, utilizing an anti-tag antibody, showed the disappearance of the tag upon successful sortase cleavage and subsequent glycan addition, indicating efficient labeling. Importantly, these experiments, performed with and without calcium ions, demonstrated the calcium dependency of the sortase-specific transpeptidase activity, confirming successful glycan labeling at the intended protein site. The successful post-labeling purification procedures are illustrated by Figure 4, which shows SDS-PAGE and Western Blot analyses of samples subjected to Size Exclusion Chromatography (SEC) for buffer exchange and enzyme removal, followed by affinity chromatography to separate labeled from unlabeled protein. This demonstrates that the inventive method enables the production of a pure and homogeneous population of glycanconjugated IgG particles.

Functional validation of the glycan-labeled IgG variants was performed through ASGPR-binding analysis. Figure 6 and Figure 5 (Normalized ELISA results highlighting relative binding differences) illustrate indirect ELISA data demonstrating the successful binding of our labeled IgG-TGN variants to the GalNAc-specific cell-surface asialoglycoprotein receptor (ASGPR). Data from conditions including EDTA strongly confirmed a glycan-specific binding of the IgG molecules to ASGPR, verifying that the labeled proteins were functional and specifically bound to GalNAc-specific lectins. Further evaluation of glycan binding via Surface Plasmon Resonance (SPR) is presented in Figure 7, which assesses the affinity and binding kinetics of our three glycan-modified IgG variants for ASGPR. The results show that higher-order modifications (e.g., labeling both heavy and light chains) lead to an increase in response units, correlating with improved efficiency and binding to the target. Figure 8 complements this by showing SPR analysis of concentration-dependent glycan binding responses, visually confirming that labeling both heavy and light chains, or the heavy chain alone, is more efficient than labeling only the light chain.

Importantly, Figure 9 demonstrates that the modification process did not compromise the antibodies' primary function. SPR experiments confirmed that all labeled antibodies retained their high affinity binding to their specific target, in this case, Siltuximab's binding toward IL-6, ensuring the preservation of their therapeutic potential.

Finally, Figure 10 provides compelling evidence of successful glycan modification through mass spectroscopic analysis. Liquid chromatography (LC) mass spectra clearly show specific mass shifts in the modified IgG variants compared to unlabeled references. The accompanying table in Figure 10 presents the precise molecular masses obtained by LC-MS in reference to calculated masses, confirming the successful sortase-mediated glycan addition and the expected increase in molecular mass at each labeling site.

## Claims

1. **A method adding a carbohydrate to a target polypeptide or to a target protein,** the method comprising reacting an acceptor protein construct with a donor carbohydrate construct in the presence of an enzyme having sortase function, wherein
(A) The acceptor protein construct has the formula:
**TP-L1-SRM-L2-DT**
wherein:
TP is a target polypeptide or target protein,
SRM is a polypeptide having an amino acid sequence comprising a sortase recognition motif, and
**DT** is absent or is a detectable tag,
**L1** is absent or a first linker,
**L2** is absent a second linker; and
(B) The donor carbohydrate construct has the formula:
**OG-L-SM**
wherein:
**OG** is Xₙ with X being an amino acid, n = 1 or more; preferably X is G, A, S, T, C or K, preferably OG is an oligoglycine,
**L** is absent or a linker
**SM** is a sugar molecule, preferably an amino sugar molecule.

2. The method of claim 1, wherein **SRM** is connected to the C-terminus of **TP** directly or via **L1.**

3. The method of claim 1 or 2, wherein the **DT** is connected to the C-terminus of **SRM** directly or via **L2.**

4. The method of any one of claims 1 to 3, wherein the target polypeptide or target protein is an antigen binding protein (ABP), wherein the ABP is any protein capable of binding specifically to, or unspecifically to, any other protein or polypeptide, such as preferably an antibody, or an antigen binding fragment thereof, nanobody, or an antigen binding fragment thereof, a soluble or membrane bound receptor, or a protein ligand.

5. The method of any one of claims 1 to 4, wherein n in Xₙ is 2 or more, preferably is 3, 4, 5, 6, 7, or 8.

6. The method of any one of claims 1 to 5, wherein the sugar is a glycan, preferably a glycan known to be present on a glycoprotein, for example the glycan may be a monosaccharide, an oligosaccharide or a polysaccharide.

7. The method of any one of claims 1 to 6, wherein **SM** is connected to the C-terminus of **OG** directly or via a linker.

8. The method of any one of claims 1 to 7, wherein a first product of the reaction has the formula:
**TP-L1-partialSRM1-OG-L-SM**
wherein
**partialSRM1** is a N-terminal sequence of the original sortase recognition motif (which lacks an original C-terminal part of the sortase recognition motif)

9. The method of any one of claims 1 to 8, wherein a second (side) product of the reaction has the formula:
**partialSRM2-L2-DT**
wherein
**partialSRM2** is a C-terminal sequence of the original sortase recognition motif (which lacks a N-terminal sequence of the sortase recognition motif).

10. **An acceptor protein construct,** comprising the formula:
**TP-L1-SRM-L2-DT**
wherein:
**TP** is a target polypeptide or target protein,
**SRM** is a polypeptide having an amino acid sequence comprising a sortase recognition motif, and
**DT** is absent or is a detectable tag,
**L1** is absent or a first linker,
**L2** is absent a second linker,
Preferably, wherein the acceptor protein construct is as defined in any one of the preceding claims.

11. **A donor carbohydrate construct,** comprising the formula:
**OG-L-SM**
wherein:
**OG** is Xₙ with n = 1 or more; preferably OG is an oligoglycine,
**L** is absent or a linker
**SM** is a sugar molecule, preferably an amino sugar molecule;
Preferably, wherein the donor carbohydrate construct is as defined in any one of claims 1 to 9.

12. **A protein sugar conjugate construct,** having the formula:
**TP-L1-partialSRM1-OG-L-SM**
Wherein
**TP** is a target polypeptide or target protein,
**partialSRM1** is a polypeptide having an amino acid sequence comprising a N-terminal part of the sortase recognition motif (lacking an C-terminal part of the sortase recognition motif)
**L1** is absent or a first linker,
**OG** is Xₙ with n = 1 or more; preferably OG is an oligoglycine,
**L** is absent or a linker.
**SM** is a sugar molecule, preferably an amino sugar molecule.

13. The protein sugar conjugate construct of claim 12, wherein the L1 is a protein linker, preferably a peptide linker, more preferably a GS repeat linker.

14. **A use** of the donor carbohydrate construct of claim 11 in the conjugation of a carbohydrate to a target protein or target polypeptide.

15. **A kit,** comprising the donor carbohydrate construct of claim 11, and, optionally, instruction for using the donor carbohydrate construct in a method of any one of claims 1 to 9.
